# EUROPEAN PATENT SPECIFICATION

(11) **EP 4 346 917 B1**
(45) Date of publication and mention of the grant of the patent: **01.07.2026**
(21) Application number: 22732047.0
(22) Date of filing: 27.05.2022
(51) Int. Cl.: A61L 2/00, A61M 25/00, A61B 90/50, A61N 5/06, A61F 13/00, A61M 25/02

(54) **SYSTEM FOR DISINFECTING SKIN TISSUE AROUND CATHETERS**
SYSTEM ZUR DESINFEKTION VON HAUTGEWEBE UM KATHETER
SYSTÈME DE DÉSINFECTION DE TISSU CUTANÉ AUTOUR DE CATHÉTERS

(30) Priority: 28.05.2021 CH 6152021
(43) Date of publication of application: 10.04.2024
(73) Proprietor: Aseptuva AG, 3008 Bern (CH)
(72) Inventor: TWERENBOLD, Henry, 3008 Bern (CH); SAIKIA, Eashan, 3008 Bern (CH); SOUZA LIMA, Rafael, 3008 Bern (CH)
(74) Representative: Keller Schneider Patent- und Markenanwälte AG
(86) International application number: PCT/EP2022/064455
(87) International publication number: WO 2022/248691

(56) References cited:
- EP-A1- 3 603 692
- WO-A1-2012/177803
- WO-A1-2014/165854
- WO-A1-2022/140633
- WO-A1-2022/170171
- US-A1- 2003 018 373
- US-A1- 2019 192 707

## Description

### FIELD OF THE INVENTION

The present invention relates to a system for disinfecting skin tissue around catheters according to the preamble of patent claims.

### BACKGROUND OF THE INVENTION

Intravenous transfusion is a widely popular technique for automated delivery of life-saving drugs and fluids directly into the patient's body. This procedure involves a catheter, with one of its ends inserted into a vein, located at an appropriate site such as the subclavian vein, jugular vein, femoral vein or peripheral vein, depending on the specific treatment. A prime example of such catheters is the Central Venous Catheter (CVC), which has several sub-types like tunneled CVCs, non-tunneled CVCs, peripherally inserted central catheters (PICC) and the implanted port. The specific choice of a CVC is determined by the physician, depending on the estimated application period, which can range from a few days to several months. However, the use of catheters is associated with a range of infections, such as bloodstream infections commonly known as Central Line Associated Bloodstream Infections (CLABSI) and Catheter-related Bloodstream Infections (CRBSI).

CLABSI are a type of Hospital Acquired Infections (HAI), that can occur both in the Intensive Care Unit (ICU), or in a non-ICU environment. Furthermore, CLABSI also occur in a non-hospital setting wherein a CVC is inserted into a patient for continual treatment such as in the family home or old-age homes. In regard to HAI, the US reports 1.7 million cases and the EU 4.2 million cases per year [World Health Organization. "Health care-associated infections fact sheet." World Health Organization 4 (2015)]. In addition to this, HAI like CLABSI often prolong hospital stays by 10 - 14 days. This leads to average additional costs of USD 30,000 per patient, which amounts to over several billions of USD in healthcare expenses every year. Given those circumstances, it is of utmost importance to not only effectively treat such a severe health problem, but also to strive to prevent the onset of such an infection.

Current recommendations by medical experts mandate an exhaustive multimodal approach [Zingg, Walter, et al. "Hospital-wide multidisciplinary, multimodal intervention programme to reduce central venous catheter-associated bloodstream infection." PloS one 9.4 (2014): e93898], consisting of several therapies to ensure a safe intravenous procedure. These therapies include careful skin preparation, catheter stabilization and securement, patient cleansing, and antibiotic treatment. Although the guidelines developed for these collective strategies have shown effectiveness, it causes an immense burden on the healthcare workers to ensure consistency, in addition to the specialized training and education needed from the workers.

Over the past few decades, different technical solutions have also emerged to counter such infections [CDC - Centers for Disease Control and Prevention. "Guidelines for the Prevention of Intravascular Catheter-Related Infections" (2011)]. One significant improvement pertains to the addition of a non-fouling coating on the inner lumen of the tubes. This technique slows down the rate of biofilm formation on the catheter surfaces, thus delaying the dwell time for CLABSI.

Another promising solution is offered by light-based therapies, which exploit the germicidal properties of light at certain wavelengths. A suitable candidate is Ultraviolet-C (UVC) radiation, which ranges between 100-280 nm, and it is known to possess a bacteriostatic effect. However, earlier studies have shown that the radiation on the prevalent commercially- applied 254 nm can trigger a carcinogenic effect [Blum, Harold F., and Stuart W. Lippincott. "Carcinogenic effectiveness of ultraviolet radiation of wavelength 2537A." Journal of the National Cancer Institute 3.2 (1942): 211-216]. Furthermore, it is also hazardous to the eye for its tendency to cause cataract in the eye, leading to blindness. Due to such problems, UVC therapy has been limited to disinfection procedures that do not involve a direct exposure to the human body.

In this context, several medical device technologies have introduced potential concepts to use UVC for disinfecting surfaces of tubes and catheters. However, most of these solutions focus on the inner lumen, thereby shielding the human tissue from direct exposure to UVC radiation.

A recent light introduction device and sterilization system (EP3603692A1) proposes a thick light-permeable member around a medical conduit. The described member allows propagation of UVC radiation, preferably between 270-340 nm, along the length of the conduit. However, this UVC wavelength falls in the range that is known to be hazardous. Furthermore, having such an external member surrounding a conduit like a CVC, will restrict the delivery of life-saving drugs and fluids, through the exit holes present along the surface of the CVC.

Previous attempts for CVC (US2018369432A1; US2019262486A1; WO2015066238) consist of further concepts that involve disinfection of the inner lumen.

Recent in-vitro studies have demonstrated that at the Far-UVC range (< 230 nm), the radiation indeed has a germicidal effect without causing damage onto living tissues [Buonanno, Manuela, et al. "Germicidal efficacy and mammalian skin safety of 222-nm UV light." Radiation research 187.4 (2017): 493-501; Buonanno, Manuela, et al. "207-nm UV light-a promising tool for safe low-cost reduction of surgical site infections. II: In-vivo safety studies." PloS one 11.6 (2016): e0138418]. An application of this wavelength is described in the application WO2016187145, which discloses a portable battery powered laser source, connected to optical fibers, in order to guide light again only into the lumen of the catheter.

In a concept from 2019, optical fibers are also incorporated longitudinally into the frame of different catheters (US20190168023A1). Besides that, light delivery is only possible to the skin underlying the catheter without targeting specifically the skin around and within the insertion site. In addition, the applied radiation should be preferably in the hazardous range of 400-500 nm wavelength. Due to this longitudinal design along the whole medical device, such catheters are constructed totally of optically transparent material. Potential side-effects for fluids and medications within the described material through light disinfection are not known.

For example, US 2003/018373 discloses a device for applying ultraviolet light to a portion of a catheter as well as near an entrance site of the catheter. The device comprises a housing enclosing a light source and a reflector. The light source may be protected by a UV transmissive window or screen. A light seal is disposed around an opening of the device, the light seal creating a substantially light-tight chamber when pressed on a patient or object. By means of the reflector, the light emitted by the light source may illuminate the target surfaces from a multitude of different angles. A reflective surface may be included on a light directing component to reflect light from the light source to a partially shadowed area.

EP 3 603 692 A1 describes a light introduction device and a sterilization system. The light introduction system includes an inner cavity allowing passage of at least one substance introduced into a human body, whereby light is introduced to a part of a medical conduit having a light permeable thick portion arranged outside of a human body. The light introduced into the light permeable thick portion of the conduit can propagate to bacteria present in a substance contacting the medical conduit. The light introduction device preferably includes a light guide member surrounding the outer surface of the medical conduit. The light thereby enters the medical conduit inclined with respect to a longitudinal direction of the medical conduit. The light preferably has a peak wavelength of 270nm to 340nm.

WO 2014/165854 discloses a disinfection device to control infections related to indwelling catheters. The device combines a component for trans-catheter wall UV light treatment to eradicate bacteria within the catheter lumen and a component for external UV light treatment to the catheter external surface and a surface of a patient's tissue surrounding the insertion site of the catheter. The disinfection device uses a wavelength of light having an antimicrobial activity, such as UVC. The device may be connected to an external light source and the light may be delivered from this external light source to the device by means of optical fibres. The light may shine towards the patient's tissue surrounding the insertion site of the catheter, for example circumferentially all around the opening of the urethra.

US 2019/0192707 A1 describes an apparatus for disinfecting any of a host of surfaces including those associated with medical insertion sites and superficial wounds of a patient using light at a wavelength that is capable of killing bacteria, such as UV light, the light being delivered by one or both of a radially emitting optical fibre and an end emitting optical fibre. A light delivery umbilical comprising one or more transport fibres may be used to transport light from a light source to one or more optical fibres disposed in one or more of a main shaft, an infusion shaft and a hub.

### SUMMARY OF THE INVENTION

It has been realized as part of the present disclosure that once a catheter, such as a CVC, has been inserted into the skin, there is a risk of developing a bacterial biofilm, both on the inner lumen and outer surface of the CVC. This biofilm is a result of different types of bacteria present in the environment. The bacteria can accumulate around the insertion site of the catheter or tube. If the CVC slides in-and-out of the insertion hole due to patient's body movements, it eventually enters the bloodstream. This may further lead to various infections, including bloodstream infections such as Central Line Associated Bloodstream Infections (CLABSI) and Catheter-related Bloodstream Infections (CRBSI).

The previously known systems for disinfecting skin tissue around catheters suffer from different drawbacks. These include poor to no disinfection of the skin tissue. Further drawbacks are adverse health consequences, such as allergic reactions, irritation, carcinogenic side-effects. A further challenge is that many pathogens present in the hospital might be resistant to treatment, such as antibiotic or antiseptic treatment, including chlorhexidine. There is also a recurrent need of intervention by the healthcare staff, which may be a further source of infection. Furthermore, known non-fouling coatings frequently only partially prevent adherence and are often not germicidal.

It is therefore the general objective of the present invention to advance the state of the art with respect to systems for disinfecting skin tissue around catheters, in particular human skin tissue. In advantageous embodiments, the disadvantages of the prior art are overcome fully or partly.

In advantageous embodiments, a system for disinfecting skin tissue is provided which is less harmful, in particular non-harmful. In advantageous embodiments, a system for disinfecting skin tissue is provided which targets all infection-prone regions including catheter and surrounding skin. In advantageous embodiments, a system for disinfecting skin tissue is provided which is free of chemicals, such as chemical disinfectants. In advantageous embodiments, a system for disinfecting skin tissue is provided which does not have adverse health effects, such as inducing allergic reactions or irritations, which may possibly be due to certain antiseptics. In advantageous embodiments, a system for disinfecting skin tissue is provided which overcomes antibiotic resistance. In advantageous embodiments, a system for disinfecting skin tissue is provided which shields the wound to pathogens, particularly external pathogens. In advantageous embodiments, a system for disinfecting skin tissue is provided which reduces the need for intervention or handling by hospital staff.

The general objective is solved by the subject-matter of the independent claim.

Advantageous embodiments follow from the dependent claims and the overall disclosure.

In a first aspect, the general objective is achieved by a system for disinfecting skin tissue around catheters, which system comprises an adaptor. The adaptor defines a catheter entry opening for entry of a catheter into the adaptor and a catheter exit opening for exit of the catheter from the adaptor. The adaptor comprises an outer surface and an inner surface. The inner surface defines an inner cavity for receiving at least a section of the catheter, wherein the inner cavity extends from the catheter entry opening to the catheter exit opening. The system further comprises at least one light source for emitting UVC light, wherein the light source is arranged such that the UVC light is emitted away from and/or within the adaptor. In some embodiments, the light source is arranged such that the UVC light is emitted away from the adaptor and/or within the adaptor. In some embodiments, the light source is arranged such that the UVC light is emitted away from the adaptor, particularly only away from the adaptor.

The system disclosed herein may be used to disinfect the skin tissue around a catheter that is inserted through the skin of a subject, such as a mammal, e.g. a human or animal, such as a human patient. The adaptor may be placed around the catheter before, during or after insertion of the catheter into the skin and the adaptor may be placed close to or directly at the human skin. The light source may then emit UVC light to the skin of the subject surrounding the catheter, which leads to disinfection of the skin. Thereby, the risk of pathogenic infections is greatly reduced. The UVC light emission may be performed intermittently or continuously. The intensity level, the duration and the time interval of the application can vary with the different catheterization processes and the state of the patient.

A catheter is a tube than can be inserted into the body. Typically, the catheter enables transfer of a liquid between the inside and the outside of the body. As an example, a catheter may be used to supply the body with a liquid, such as a drug.

For example, the catheter is inserted through the human skin. As an example, the catheter may be a venous catheter, such as a central venous catheter. In some embodiments, the skin tissue is human skin tissue, particularly the epidermis and/or dermis.

In some embodiments, the system for disinfecting skin tissue around catheters disclosed herein is a device for disinfecting skin tissue around catheters and the adaptor is a device body.

In some embodiments, the catheter entry opening and the catheter exit opening each have an opening cross section that corresponds to the cross section of the catheter. In some embodiments, the catheter entry opening and the catheter exit opening may each be circular, particularly with a diameter from 0.1 mm to 30 mm, preferably from 0.3 mm to 15 mm, more preferably from 0.5 mm to 12 mm, more preferably from 1 mm to 11.3 mm. In some embodiments, the catheter entry opening and the catheter exit opening may each be circular, in particular with a diameter from 1 mm to 5 mm, such as from 2 mm to 3 mm.

In some embodiments, the inner surface of the adaptor defines an inner cavity for receiving and guiding at least a section of the catheter. As an example, the inner surface of the adaptor may be complementary to the surface of the catheter. One advantage of this is that the catheter is securely held by the adaptor. In further embodiments, the inner cavity may be essentially tubular, e.g. with a diameter from 1 mm to 20 mm, particularly from 1.2 mm to 12 mm. In some embodiments, the inner cavity extends essentially longitudinally from the catheter entry opening to the catheter exit opening.

UVC light is defined as light with a wavelength in the range from 100 nm to 280 nm. In some embodiments, the UVC light is in the range from 180 nm to 260 nm, particularly from 180 nm to 235 nm, more particularly from 200 nm to 230 nm, more particularly 220 nm ± 5 nm. In some embodiments, the light source is configured such that the UVC light is emitted away from the adaptor, particularly away from the outer surface of the adaptor. In some embodiments, the light sources are arranged such that the UVC light is emitted away from the adaptor, particularly away from the outer surface of the adaptor, at a light emitting angle of 45° - 135°, particularly 80° - 100°, more particularly 90°. The light emitting angle is the angle at which the UVC light is emitted away from the adaptor. In particular, the light emitting angle is measured relative to the outer surface that surrounds the light source. As an example, a light emitting angle of 90° means that the UVC light is emitted perpendicularly away from the adaptor. In some embodiments, the light sources are configured to emit the UVC light at a cone angle of up to 170°, such as up to 120°, such as up to 90°.

In some embodiments, at least one of the at least one light sources is arranged on the outer surface of the adaptor. In some embodiments, the at least one light source, particularly all light sources, is arranged on the outer surface of the adaptor. In some embodiments, no light source is arranged on the inner surface of the adaptor. In other words, in these embodiments, the inner surface of the adaptor is free from light sources. In further embodiments, the light sources are arranged such that less than 5%, preferably less than 2%, more preferably less than 1%, even more preferably 0%, of the emitted UVC light is emitted towards the inner surface of the adaptor. In some embodiments, the at least one light source is arranged such that the UVC light that is emitted from the at least one light source is not emitted towards the inner cavity of the adaptor. One advantage of these embodiments is that a catheter that is placed inside the adaptor is not exposed to UVC radiation, which may cause harm or damages to the catheter. As a result, these embodiments create a mild environment for the catheter and enable the use of a broad range of different catheters, including ones which are susceptible to damages caused by UVC radiation. A further advantage of these embodiments is that the UVC radiation is concentrated to the skin tissue around the catheter, which leads to a high energy efficiency.

According to the invention, the light source is configured for emitting UVC light away from and/or within the adaptor. In some embodiments, the light source is configured for emitting UVC light away from the adaptor. In some embodiments, the light source is configured for emitting UVC light only away from the adaptor.

In some embodiments, the light source is configured such that, when a catheter is inserted into the system such that an outer section of the catheter is arranged outside the system and protrudes from the catheter exit opening, the outer section of the catheter is irradiated with the UVC light emitted from the light source. In some embodiments, the outer section of the catheter has a length from 1 mm to 100 mm, preferably from 5 mm to 30 mm.

In some embodiments, the inner surface of the adaptor is coated with a coating, particularly with an antimicrobial coating, such as an antibacterial and/or antiviral coating. Preferably, in these embodiments, the inner surface of the adaptor is configured such that the coating is in physical contact with the catheter around which the adaptor is placed. In some embodiments, the outer surface of the adapter comprises a front outer surface, as described in further detail below. In some embodiments, the front outer surface of the adaptor is coated with a coating, particularly with an antimicrobial coating, such as an antibacterial and/or antiviral coating. The coating may, for example, comprise chlorhexidine, silver sulfadiazine, minocycline rifampin, povidone iodine antiseptic ointment or bacitracin / gramicidin / polymyxin B ointment, or any combination thereof. The inner surface of the adaptor and/or the front outer surface of the adapter may, for example, be coated with the coating by way of a patch, such as an adhesive patch. The system may therefore further comprise an adhesive patch comprising the coating. The adhesive patch may have a shape that is identical to the front outer surface of the adaptor.

One advantage of these embodiments is that microorganisms, particularly pathogens such as viruses or bacteria, that are located on the outer surface of the catheter or on the system, such as the adaptor, and may otherwise enter the body through the catheter, are killed, which reduces the risk of diseases associated with the use of catheters, such as hospital acquired infections.

In some embodiments, the at least one light source is at least partially arranged adjacent to, in particular around, the catheter exit opening and is configured such that UVC light can be emitted away from the adaptor, particularly away from the outer surface of the adaptor. At least partially, in this context, means that, where the system comprises more than one light source for emitting UVC light, at least one of the light sources is arranged adjacent to, in particular around, the catheter exit opening. In some embodiments, when the adapter is placed around a catheter, the UVC light is emitted away from the adaptor, in the direction of the catheter exiting the adaptor. In some embodiments, the at least one light source is arranged within 10 cm, particularly within 5 cm, more particularly within 1 cm, from the catheter exit opening. In some embodiments, the at least one light source is arranged within a ring surrounding the catheter exit opening and having a radius of 10 cm, particularly 5 cm, more particularly 1 cm. In some embodiments, the at least one light source is arranged within a ring surrounding the catheter exit opening, wherein the ring has a circular outer circumference from 6 mm to 160 mm, particularly from 9 mm to 80 mm. In some embodiments, the ring has a diameter from 2 mm to 50 mm, particularly from 3 mm to 25 mm. In some embodiments, the catheter exit opening is arranged centrally within the ring surrounding the catheter exit opening. In some embodiments, the center point of the ring surrounding the catheter exit opening lies on the center point of the catheter exit opening. As an example, if the catheter exit opening is circular, the center point of the catheter exit opening may be identical with the center point of the ring surrounding the catheter exit opening. One advantage of these embodiments is that they ensure that the UVC light is emitted selectively into those areas of the skin that surround the catheter. Since these areas of the skin are particularly vulnerable and prone to infection, these embodiments efficiently reduce the overall risk of infection.

In some embodiments, the light sources, such as the light emitting openings of the one or more light guides, are distributed evenly around the catheter exit opening. The even distribution contributes to an even radiation of the skin around the catheter, which enhances the fidelity of the disinfection.

In some embodiments, the at least one light source includes one or more of the following: a light-emitting diode (LED), a laser, a flash lamp, an excimer, an arc lamp, a field emission source, a wide band semiconductor, and a photo-excited emission source, such as a UV phosphor. The field emission source may, for example, be a field emission lamp, such as a (cathode) luminescent phosphor. In some embodiments, all light sources are LEDs. One advantage of using LEDs is that large areas may be illuminated.

In some embodiments, the at least one light source is suitable for emitting UVC light at a radiant flux of at least 100 µW, preferably from 400 µW to 25000 µW.

According to the invention, the system further comprises a light guide which is configured for guiding UVC light from a light receiving opening of the light guide to at least one light emitting opening of the light guide, wherein the at least one light source includes a light emitting opening of the light guide. A light guide is a fiber, such as an optical fiber, which is suitable for guiding light. In some embodiments, the light guide comprises a light emitting end section defining the light emitting opening. In some embodiments, the light emitting end section has a length from 0.5 mm to 30 mm, such as from 1 mm to 15 mm, wherein the length extends along a longitudinal direction of the light guide.

In some embodiments, the light emitting end section of the light guide comprises a beam expander for expanding the cross section of the emitted UVC light, such that the emitted UVC light preferably has an illumination diameter of up to 4 cm², preferably from 1 cm² to 4 cm². One advantage of these embodiments is that they enable a large area of skin to be irradiated.

In some embodiments, the light emitting end section tapers towards the light emitting opening of the light guide. In other words, in these embodiments, the cross section of the light emitting end section decreases towards the light emitting opening. One advantage of this is that it decreases the light spot size and increases divergence.

In some embodiments, the cross section increases, in particular continuously increases, towards the light emitting opening. In other words, in these embodiments, the light emitting end section is funnel-shaped. One advantage of these embodiments is that the light spot size is increased and the intensity density of the emitted light is decreased.

In some embodiments, the light emitting end section is spherical. One advantage of this is that the light collection angle is increased.

In some embodiments, the light emitting end section comprises a lens. One advantage of this is that the divergence of the emitted light is increased and the illuminated area is increased.

In some embodiments, the light emitting end section has a spherical cross section. In some embodiments, the light emitting end section comprises a diffuser. One advantage of this embodiment is that the light emitted from the light emitting opening is already diffused, thus illuminating a large area of skin.

A light guide comprises a first end and a second end. Preferably, the light receiving opening is arranged on the first end of the light guide and the light emitting opening is arranged on the second end of the light guide. Optionally, the light guide additionally includes light emitting openings arranged between the first and second end. A light guide which additionally includes light emitting openings arranged between its ends may be labelled side-emitting. A light guide which includes a light emitting opening that is arranged on one of the ends of the light guide may be labelled end-emitting. Side emission may, for example, be achieved by micro engravings the light guide.

In some embodiments, the light guide comprises a third end and may further comprise a fourth end and, optionally, even more ends. As an example, a light guide may include a light beam spread point connecting the first end, the second end and the third end. For example, light may enter the light guide through the first end, may pass through the light guide up to the light beam spread point, and may be spread into a first beam portion passing to the second end of the light guide and a second beam portion passing to the third end of the light guide. It is understood that the light guide may additionally comprise further light beam spread points and further ends. One advantage of these embodiments is that including light beam spread points reduces the overall amount, volume and weight of light guides, which reduces the weight and volume.

One advantage of the use of light guides is that they avoid the risks associated with electric components, such as shock or heat near the vulnerable skin tissue. In some embodiments, the light guides include optical fibers. Furthermore, examples of light guides include liquid light guides and solarization-resistant light guides, such as solarization-resistant optical fibers.

In some embodiments, the system further comprises at least one emission chamber which comprises at least one of the at least one light sources and optionally further comprises a filter and/or a diffuser. In these embodiments, the filter and/or the diffuser are preferably arranged such that the UVC light emitted from the light source passes through the filter and/or diffuser. In some embodiments, the filter is configured for selectively filtering light with a wavelength outside the UVC range. In other words, in these embodiments, only UVC light may pass through the filter. This ensures that the skin is only exposed to UVC light with wavelengths in a desired range of interest. Once again, this ensures that the damages to the skin caused by radiation are minimized. In some embodiments, the diffuser is configured to scatter the UVC light such that diffused UVC light is emitted to the skin around the catheter. In some embodiments, the diffuser includes a fused silica glass or a magnesium fluoride glass, wherein the glass surface is preferably ground, etched or frosted. One advantage of the diffuser is that the skin around the catheter is irradiated evenly with UVC radiation. In other words, ideally, the entire surface area of the skin around the catheter is subjected to UVC radiation. This contributes to a high reliability and reproducibility and further reduces the risk of infections.

According to the invention, the system further comprises an emission chamber. The emission chamber comprises at least one of the at least one light source. The emission chamber comprises the light emitting opening of at least one light guide. In some embodiments, the emission chamber comprises the at least one light source, e.g. all light sources. In a typical embodiment, the at least one light source is arranged inside the emission chamber. In some embodiments, the at least one light source is arranged such that UVC light can be emitted from the emission chamber. In some embodiments, the emission chamber comprises a filter, which is arranged inside the emission chamber. In some embodiments, the filter is arranged such that the UVC light emitted from the light source passes through the filter. In some embodiments, the emission chamber comprises a diffuser, which is arranged inside the emission chamber. In some embodiments, the diffuser is arranged such that the UVC light emitted from the light source passes through the diffuser.

According to the invention, the at least one emission chamber forms part of the outer surface of the adaptor, preferably of the front outer surface of the adaptor. The surface of the emission chamber that forms part of the outer surface of the adaptor is defined as a front exterior surface of the emission chamber. The front exterior surface of the emission chamber may, for example, have an area from 1 mm² to 4 cm², preferably from 3 mm² to 2 cm². In some embodiments, the front exterior surface of the emission chamber is rectangular. In some embodiments, the front exterior surface of the emission chamber is circular. In some embodiments, the emission chamber has a thickness from 1 mm to 30 mm, preferably from 5 mm to 15 mm. The thickness preferably extends orthogonal to the front exterior surface of the emission chamber. The thickness may also extend along the inner cavity.

In some embodiments, the emission chamber has an inner surface, which is at least partially, preferably fully, covered with a scattering layer configured to scatter and reflect UVC light. In some embodiments, the scattering layer is configured to reflect at least at least 50%, particularly at least 60%, more particularly at least 70%, more particularly at least 80%, more particularly at least 90%, more particularly at least 95%, more particularly at least 98%, more particularly 100%, of incident UVC light. In some embodiments, the scattering layer is configured to scatter at least at least 50%, particularly at least 60%, more particularly at least 70%, more particularly at least 80%, more particularly at least 90%, more particularly at least 95%, more particularly at least 98%, more particularly 100%, of the incident UVC light. In some embodiments, at least 50%, particularly at least 60%, more particularly at least 70%, more particularly at least 80%, more particularly at least 90%, of the inner surface of the emission chamber is covered with the scattering layer. In some embodiments, the scattering layer may comprise, e.g. be made of, aluminum. As a further example, the scattering layer may comprise, preferably be made of, polytetrafluoroethylene (PTFE). As a further example, the scattering layer may comprise, e.g. be made of, UV phosphor. The UV phosphor may, for example, be activated by an electron beam. In some embodiments, at least the inner surface of the emission chamber that is closest to the rear outer surface of the adaptor is covered with a scattering layer. One advantage of these embodiments is that they increase the intensity of the UVC light emitted from the emission chamber by scattering light beams that would otherwise not be reflected but instead be absorbed. In some embodiments, the scattering layer is arranged opposite the filter and/or the diffuser. One advantage of using a scattering layer made of PTFE is that it may be used with an MRI procedure as it does not interfere with the MRI procedure. One further advantage of PTFE is that it has higher reflectivity than aluminum.

In some embodiments, the at least one emission chamber is arranged within 1 mm to 50 mm, preferably 1 mm to 20 mm, from the catheter exit opening.

In some embodiments, the system comprises at least two, preferably from two to ten, more preferably from four to eight emission chambers. In some embodiments, each emission chamber comprises from one to 15, preferably from one to six, more preferably one light source. The light source may, for example, be an LED or a light emitting opening of a light guide. In some embodiments, the emission chambers are arranged within 1 mm to 50 mm, preferably 1 mm to 20 mm, from the catheter exit opening. In some embodiments, the emission chambers each have the same distance from the catheter exit opening and from each other. In other words, the emission chambers may be arranged in a symmetrical fashion around the catheter exit opening. One advantage of these embodiments is that they enable a uniform irradiation of the skin. As an example, the emission chambers may be arranged in a hexagonal fashion around the catheter exit opening.

In some embodiments, the system comprises one emission chamber. In some embodiments, the one emission chamber comprises from three to 15, particularly from four to eight, such as six, light sources. The light sources may, for example, include LEDs or light emitting openings of light guides. In some embodiments, the emission chamber surrounds the catheter exit opening. In some embodiments, the emission chamber is arranged within 1 mm to 50 mm, preferably 1 mm to 20 mm, from the catheter exit opening. One advantage of these embodiments is that they enable a uniform irradiation of the skin. In some embodiments, the catheter exit opening forms part of the emission chamber. As an example, the catheter may extend through the emission chamber.

In some embodiments, the emission chamber may be flush with the surrounding outer surface of the adaptor. As an example, the front exterior surface of the adaptor may be flush with the surrounding outer surface of the adaptor. One advantage of these embodiments is that the system has a smooth outer surface which may securely rest on, e.g., the skin of a patient. Furthermore, these embodiments contribute to an efficient and focused emission of UVC radiation only to the skin around that catheter, and not to other parts of the skin, which increases the efficiency and minimizes the overall risk of damages caused to the skin by radiation. In some embodiments, the filter and/or the diffuser form part of the outer surface of the adapter. As an example, the filter and/or the diffuser may enclose the emission chamber.

In some embodiments, the outer surface of the adaptor defines a recess located around the catheter exit opening. The emission chamber or the at least one light source may be arranged in the recess. One advantage of these embodiments is that by being arranged in the recess, the at least one light source is protected from damage. A further advantage is that the recess focusses the UVC light emitted from the light source and thus limits the area of skin that is exposed to UVC radiation, which reduces the overall risk of skin damage caused by UVC radiation.

In some embodiments, the outer surface of the adaptor comprises a front outer surface surrounding the catheter exit opening, a rear outer surface opposite the front outer surface and a side outer surface arranged between the front outer surface and the rear outer surface. In some of these embodiments, the light source is configured such that light is emitted away from the front outer surface.

The front outer surface and the rear outer surface may, for example, have a similar shape and/or size. As an example, the front outer surface and/or the rear outer surface may be oval, such as circular or elliptic. The front outer surface and/or the rear outer surface may have a surface area from 0.25 cm2 to 20 cm2, such as from 0.5 cm2 to 12 cm2. In some embodiments, the front outer surface and the rear outer surface are essentially parallel to each other. In some embodiments, the rear outer surface surrounds the catheter entry opening.

In some embodiments, the adaptor has a volume from 0.05 cm3 to 40 cm3, particularly from 0.15 cm3 to 15 cm3.

In some embodiments, the side outer surface is a mantle surface. In some embodiments, the side outer surface surrounds the adaptor circumferentially. In some embodiments, the side outer surface has a surface area from 0.02 cm2 to 18 cm2, particularly from 0.21 cm2 to 10 cm2. In some embodiments, the front outer surface and the rear outer surface are separated by an adaptor height from 1 mm to 25 mm, particularly from 3 mm to 10 mm. The adaptor height may correspond to a height of the side outer surface. In some embodiments, the ratio of the surface area of the front outer surface and the rear outer surface is between 0.5:1 and 1:0.5, preferably 1:1.

In some embodiments, the front outer surface is essentially planar and suitable for resting the system on the skin, particularly directly on the skin. This contributes to a controlled radiation of the skin and thus minimizes the risk of skin damage caused by excessive UVC radiation.

According to the invention, the emission chamber forms part of the front outer surface of the adaptor and is arranged around the catheter exit opening. These features enable the regioselective irradiation of only those parts of the skin that surround the catheter that was, is or will be inserted into the skin. This ensures that the overall risk of skin damage associated with UVC radiation is reduced.

In some embodiments, at least 50%, such as at least 60%, particularly at least 70%, more particularly at least 80%, even more particularly 90%, preferably all emission chambers, form part of the front outer surface of the adaptor. In some embodiments, at least 50%, such as at least 60%, particularly at least 70%, more particularly at least 80%, even more particularly 90%, preferably all light sources form part of the front outer surface of the adaptor. The light sources may include one or more light emitting openings of at least one light guide and/or one or more LEDs.

In some embodiments, the system comprises 1-20, preferably 3-7, such as 5, light guides, each light guide having at least one, preferably multiple, light emitting openings. The light guides may be end-emitting and may optionally additionally be side-emitting. In some embodiments, each light guide comprises an external section arranged outside the adaptor and an internal section arranged inside the adaptor. In some embodiments, the adaptor comprises a light guide entry opening for entry of the light guide into the adaptor. In some embodiments, the light guide entry opening is arranged in the side outer surface of the adaptor. The light guides may be at least partially bundled together to form one or more light guide bundles. In some embodiments, the external sections of the one or more light guides are bundled together to form a light guide bundle. The internal sections of the one or more light guides may or may not be bundled. The light guide bundles reduce the number of lose light guides and thus render the system more practical for handling and for use.

In some embodiments, the light guides are solarization-resistant. Solarization-resistant means that the transmission of UVC light is increased and the material degradation induced by UV exposure is reduced. Solarization resistance may be achieved by high -OH fused silica coating of the fiber core.

In some embodiments, the adaptor comprises, particularly consists of, a first adaptor portion and a second adaptor portion which are releasably connectable to each other, preferably in a form-fit fashion. Releasably, in this context, means that the structural integrity of the first adaptor portion and the second adaptor portion is not destroyed. It includes multiple connection and disconnection cycles. In a typical embodiment, the first adaptor portion and the second adaptor portion are detachable. In some embodiments, the first adaptor portion and the second adaptor portion each have a connecting surface that intersects, particularly bisects, the cavity of the adaptor, such that the first adaptor portion comprises a first section of the inner surface of the adaptor and the second adaptor portion comprises a second section of the inner surface of the adaptor. The first section of the inner surface of the adaptor and the second section of the inner surface of the adaptor may have a complementary shape. In some embodiments, the first adaptor portion and the second adaptor portion each have a connecting surface that intersects, particularly bisects, the cavity of the adaptor longitudinally. Longitudinally refers to the longitudinal direction of the cavity, which extends from the catheter entry opening to the catheter exit opening. In some embodiments, the cavity is essentially tubular and the longitudinal direction refers to the direction along which the tube extends, i.e. the length of the tube.

These embodiments facilitate placement of the adaptor around the catheter. In particular, they make possible that the adaptor can be placed around a catheter after the catheter has already been inserted into the skin of the patient. The form-fit fashion enhances the stability of the system after placement around the catheter and thus enhance the security and fidelity with which the skin around the catheter may be disinfected.

In some embodiments, the adaptor is made of a material which is impermeable for UVC radiation. Impermeable, in this context, means that the transmission of UVC radiation is less than 10%, particularly less than 5%, more particularly less than 1%, more particularly 0%. As an example, the adaptor may be made of a polymer, particularly a polymer that is opaque to deep UV light. These embodiments ensure that only those sections of the skin that are around the catheter are irradiated and that other sections of the skin are not subjected to UVC radiation. As a result, the overall risk of UVC radiation-associated skin damages is reduced.

In some embodiments, the system, particularly the adaptor, is essentially tubular. It may have a diameter from 2 mm to 50 mm, particularly from 2 mm to 30 mm, more particularly from 2 mm to 12 mm. The shape may be tapered towards the front outer surface. One advantage of these embodiments is that they facilitate insertion of the system, particularly the adaptor, into the skin, such as the epidermis or, optionally, the dermis.

In some embodiments, the system comprises 1-20, preferably 3-7, such as 5, light sources. The light sources may include one or more of the following: LEDs and/or light guides, particularly light emitting openings of one or more light guides. As an example, the system may comprise 1-7, particularly 3 LEDs. The system may, alternatively or additionally, comprise 1-20, preferably 3-7, such as 5, light guides. The light guides may comprise at least one, such as 1-20 light emitting openings. In some embodiments, the total number of light sources is less than 20, such as less than 10, such as 7. One advantage of using seven light sources is that seven corresponding light guides can be efficiently packed in a hexagonal configuration.

These embodiments were found to strike an advantageous balance between providing sufficient UVC radiation for effective disinfection of the skin around a catheter, and not providing excessive UVC radiation, which would increase the risk of UVC radiation-related skin damage.

In some embodiments, the system further comprises a UVC light generator which is in optical communication with the light receiving opening of at least one light guide. The UVC light generator preferably comprises a laser and/or an excimer. In some embodiments, the light generator is connected with the light receiving opening of the at least one light guide. In some embodiments, the light generator generates UVC light, preferably only UVC light. In some embodiments, the light generator is portable. This renders the system more practical. As an example, the light generator may be attached to or positional at a patient's bed or other medical infrastructure.

In some embodiments, the system comprises a guide element for securing and guiding the catheter and, optionally, the at least one optical guide. The guide element is preferably separate from the adaptor. The guide element comprises an essentially planar resting surface for resting the guide element on the skin, and a ring-shaped jacket for securing the catheter, wherein the ring-shaped jacket is openable. Preferably, the ring-shaped jacket is releasably openable. The guide element enhances the fixation of the catheter on the skin and thus contributes to controlled disinfection of the skin.

The general objective is achieved in a second aspect of the invention by a catheter assembly comprising a catheter and a system as disclosed herein, in particular with respect to the first aspect of the invention. The catheter is inserted into the adaptor of the system as disclosed herein, in particular with respect to the first aspect of the invention.

The general objective is achieved in a third aspect, which is not part of the invention, by a method of disinfecting skin tissue around a catheter inserted into the skin of a patient. The method comprises mounting the system disclosed herein, in particular with respect to the first aspect of the invention, to the catheter. The method further comprises, subsequently, positioning the system such that the outer surface of the adaptor, preferably the front outer surface, is within 2 cm of, preferably within 1 cm of, more preferably in contact with, the skin tissue around the catheter. The method further comprises, subsequently, irradiating the skin tissue around the catheter with UVC light emitted from the at least one light source. Mounting of the system may, for example, comprise inserting the catheter into the system, particularly into the adaptor disclosed herein.

In some embodiments, the method further comprises the step of providing a catheter as disclosed herein.

In some embodiments, the skin tissue is irradiated intermittently or constantly. In some embodiments, the skin tissue is irradiated for 0.01 seconds - 60 minutes per application.

In some embodiments, the skin tissue around the catheter is irradiated with UVC light emitted through the light emitting opening of at least one light guide.

In some embodiments, the UVC light emitted from the at least one light source, particularly from the at least one light emitting opening of the at least one light guide, is emitted at a radiant flux of at least 100 µW, preferably from 400 µW to 25000 µW. The indicated ranges refer to the radiant flux of the emitted light.

The general objective is achieved in a fourth aspect, which is not part of the invention, by use of a catheter in a system, in particular with respect to the first aspect of the invention as disclosed herein. In some embodiments, the use of the catheter is ex vivo. Further disclosed is, according to a fifth aspect, a system consisting of an adaptor with light guides for in situ disinfection of skin tissue around catheters and tubes using UVC light.

In some embodiments, the adaptor can be placed around the catheter close to or directly at the human skin.

In some embodiments, the adaptor can be placed and inserted around the catheter in the epidermis layer within the perforation site.

In some embodiments, the light guides are configured to emit light to target the exterior area of tissues around the insertion site. In some embodiments, the light guides can target the epidermis layer within the insertion site. In some embodiments, the light guides can target the outer wall of the catheter. In some embodiments, the light guides can be optical fibers built in the adaptor. In some embodiments, the light guides can be optical fibers attachable to the adaptor. In some embodiments, the light guides can be solarization resistant optical fibers. In some embodiments, the light guides can be side-emitting optical fibers and/or end-tip-emitting optical fibers.

In some embodiments, the light guides can be supplemented by in situ light-emitting resources such as LEDs, photo-excited emissions or chemical reactions.

In some embodiments, the UVC light is within the wavelengths 200 nm and 230 nm, preferably at a narrow-band around the wavelength 220 nm.

In some embodiments, the system can be optically coupled to an UVC light source by means of additional light guides.

In some embodiments, the UVC light source can be connected simultaneously to several adaptors via light guides for the disinfection of multiple catheters or tubes.

In some embodiments, the UVC light source can be either a laser, an excimer or has an incorporated filter for selecting the desired wavelength.

In some embodiments, the UVC light source can be attached to the wall, to the patient's bed or to other medical infrastructure.

In some embodiments, the UVC light source can be programmed with an electronic or mechanic device for therapy-adaptability in terms of parameters such as light intensity and time duration. This device can be linked to a micro-controller-based interface for usability and data collection.

In some embodiments, the adaptor consists of non-transparent outer layer material in UVC to restrict and control the area of the disinfection process.

In some embodiments, the adaptor can consist of multiple parts that will be assembled via a coupling mechanism.

In some embodiments, the adaptor can be fixed in place by means of a bandage, a plaster or a strap-on around the body or limbs.

The adaptor is constructed for medical catheters and tubes that are inserted into the patient for different purposes such as intravenous delivery of drugs, chest drainage, dialysis and air ventilation among others. Within the adaptor, light guides such as optical fibers are placed that target in situ, directly the infection-prone sites. This refers to the skin around and within the insertion hole by shining Far-UVC light (200 nm - 230 nm) for disinfection. Those optical fibers can deliver light by end-point-emitting or side-emitting designs.

In some embodiments, the adaptor itself is placed around the catheter before, during or after the insertion process. A coupling mechanism closes the adaptor around the catheter. Eventually, it will be fixed and placed close to or directly at the human skin.

The UVC light source, such as a laser or excimer among others, may be fixed at a bed or other infrastructure and may emit light in specific intervals through light guides to the adaptor.

The intensity level, the duration and the time interval of the application can vary with the different catherization processes and the state of the patient. Once it is coupled and the light guides in the adaptor are connected to the UVC source, the disinfection may be performed automatically or manually.

Although medical catheters and tubes that are inserted into a patient, e.g. for intravenous delivery of drugs, are essential life-saving devices, they are also potentially damaging to the patient's health. The reason being that common pathogens can form a biofilm on the device's surface and on the human skin around the insertion point of these catheters and tubes, thereby increasing the risk of infection. In some embodiments, the system is a disinfection device which may be used as an add-on adaptor for plug-and-play application onto catheters and tubes that shine and target in situ Far-UVC light (200 nm - 230 nm) through light guides such as optical fibers on the infection-prone site of the human skin. The disclosure primarily concerns intravenous catheters including peripherally inserted central catheters (PICC), but it can also be implemented for other medical catheters and tubes such as chest drainage tubes, indwelling pleural catheters (IPCs), urinary tract catheters, long-term and short-term hemodialysis catheters, peritoneal dialysis catheters, external ventricular drainage (EVD) tube, endotracheal tubes, diabetes procedures and artificial stoma procedures, among others.

It is to be understood that both the foregoing general description and the following detailed description present embodiments, and are intended to provide an overview or framework for understanding the nature and character of the disclosure.

The accompanying drawings are included to provide a further understanding, and are incorporated into and constitute a part of this specification. The drawings illustrate various embodiments, and together with the description serve to explain the principles and operation of the concepts disclosed.

### BRIEF DESCRIPTION OF THE DRAWINGS

The invention described herein will be more fully understood from the detailed description given herein below and the accompanying drawings which should not be considered limiting to the invention described in the appended claims. The drawings show:
- Fig. 1: shows embodiments (Fig. 1a - 1f) of a system not belonging to the present invention featuring light guides;
- Fig. 2: shows different embodiments of a system not belonging to the present invention featuring a diffuser (Fig. 2a-2e) and embodiments according to the present invention comprising an emission chamber (Fig. 2f-2h);
- Fig. 3: shows embodiments (Fig. 3a-3c) of the system disclosed herein featuring a filter and an antimicrobial coating;
- Fig. 4: shows embodiments (Fig. 4a-4c) of a system not belonging to the present invention featuring LEDs
- Fig. 5: shows a front (Fig. 5a) and side perspective view (Fig. 5b) of an exemplary embodiment of an adaptor with in-built optical fibers, which does not belong to the present invention;
- Fig. 6: shows a side perspective view of an exemplary embodiment of an adaptor with a first version of in-built optical fibers; which does not belong to the present invention;
- Fig. 7: shows a side perspective view of an exemplary embodiment of an adaptor with a second version of in-built optical fibers, which does not belong to the present invention;
- Fig. 8: shows a side perspective view of an exemplary embodiment of an adaptor with a third version of coiled in-built optical fibers, which does not belong to the present invention;
- Fig. 9: shows, a side perspective view of an exemplary embodiment of an adaptor that shows a coupling mechanism, which does not belong to the present invention;
- Fig. 10: shows a front, side perspective view of an exemplary embodiment of a second version adaptor with in-built optical fibers in short distance to the skin, which does not belong to the present invention;
- Fig. 11: shows a front view of an exemplary embodiment of a second version adaptor with in-built optical fibers in short distance to the skin, which does not belong to the present invention;
- Fig. 12: shows a front, side perspective view of an exemplary embodiment of an insertion unit in the insertion hole with in-built optical fibers, which does not belong to the present invention;
- Fig. 13: shows a front perspective view of an exemplary embodiment of an adaptor with inbuilt optical fibers for a chest drainage tube, which does not belong to the present invention;
- Fig. 14: shows a front perspective view of an exemplary embodiment of an adaptor for an external ventricular drainage tube which is inserted into the skull of a patient, which does not belong to the present invention.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

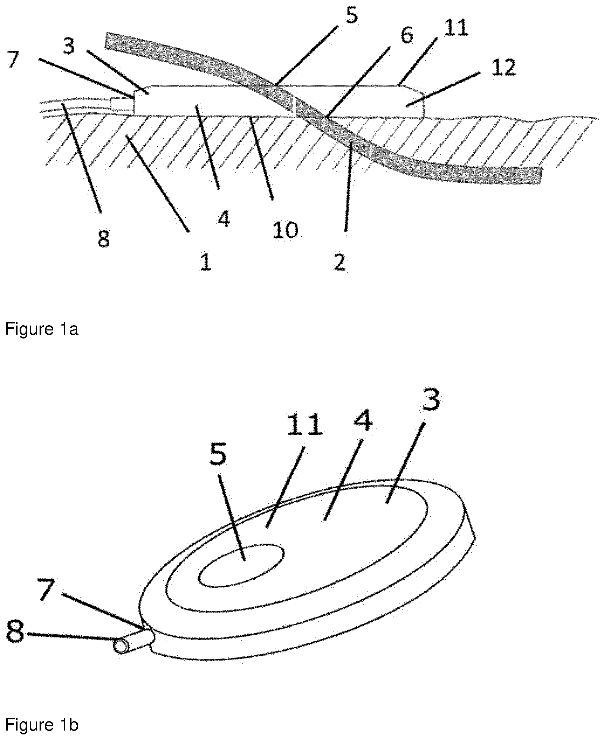

Figures 1a-1e show an embodiment of a system 3 not belonging to the present invention. The system 3 comprises an adaptor 4 that is placed around a catheter 2, which is inserted in the skin 1 of a human patient. The adaptor 4 rests on the skin 1. The adaptor 4 comprises a catheter entry opening 5 and a catheter exit opening 6 through which the catheter 2 enters and exits the adaptor 4, respectively. The system 3 further comprises a light guide 8, which enters the adaptor 4 through a light guide entry opening 7. The adaptor 4 illustrated in Fig. 1a comprises an inner surface, which is not visible in Fig. 1a, and an outer surface. The outer surface comprises a front outer surface 10, a rear outer surface 11 opposite the front outer surface 10 and a side outer surface 12 between the front outer surface 10 and the rear outer surface 11.

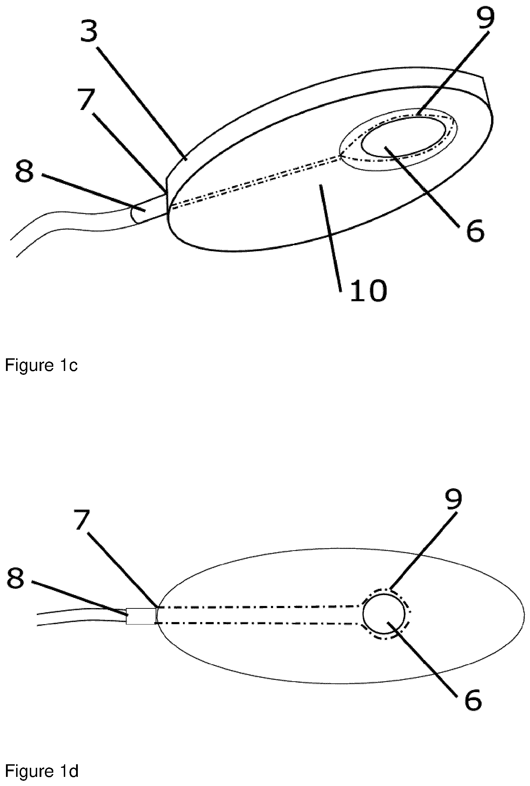

Figures 1b, 1c and 1d show the same embodiment shown in Figure 1a but from a different perspective. In particular, Figure 1b illustrates the rear outer surface 11 which surrounds the catheter entry opening 5 and Figures 1c and 1d illustrate the front outer surface 10 which surrounds the catheter exit opening 6. Figures 1c and 1d also show that the illustrated embodiment of the system 3, which is not according to the present invention, further includes a light guide 8 which includes a number of light emitting openings. The light emitting openings act as light sources 9 for emitting UVC light. In the illustrated embodiment, the light emitting elements all form part of the front outer surface 10, such that the UVC light is emitted from the light sources 9 directly to the skin 1 around the catheter 2. In the illustrated embodiment, the catheter exit opening 6 has a diameter of 5 mm and the front outer surface 10 is elliptical with a height of 2 cm and a width of 1.5 cm. In other embodiments, the front outer surface may be almost entirely capable of emitting UVC light.

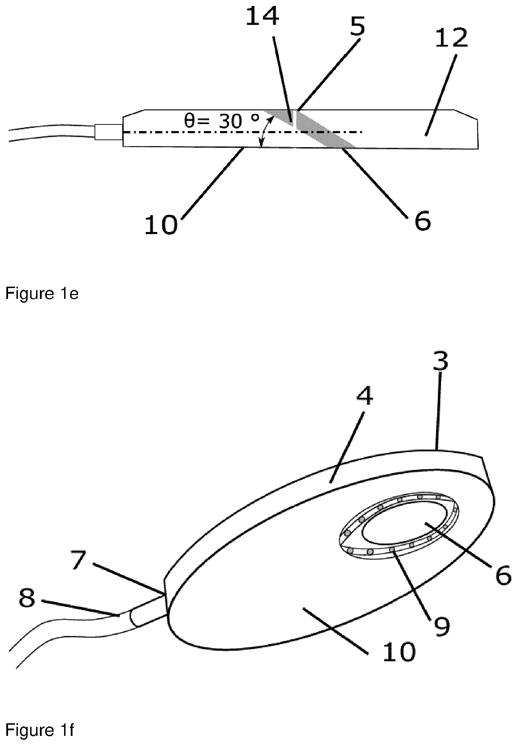

Figure 1e shows the embodiment from the same perspective as Figure 1a but does not show the catheter nor the skin 1. Instead, Figure 1d illustrates the side outer surface 12 and the cavity 14, which would normally be hidden behind the side outer surface 12 from this perspective. In the illustrated embodiment, the cavity 14 defined by the inner surface of the adaptor has a tubular shape and extends from the catheter entry opening 5 to the catheter exit opening 6. Further, the inner cavity is arranged at an angle of 30° relative to the front outer surface 10.

Figure 1f shows a similar embodiment of the system 3 to the one shown in Fig. 1d, which does not belong to the present invention. In the embodiment illustrated in Fig. 1f, the light guide 8 comprises a number of discrete light emitting openings that act as light sources 9.

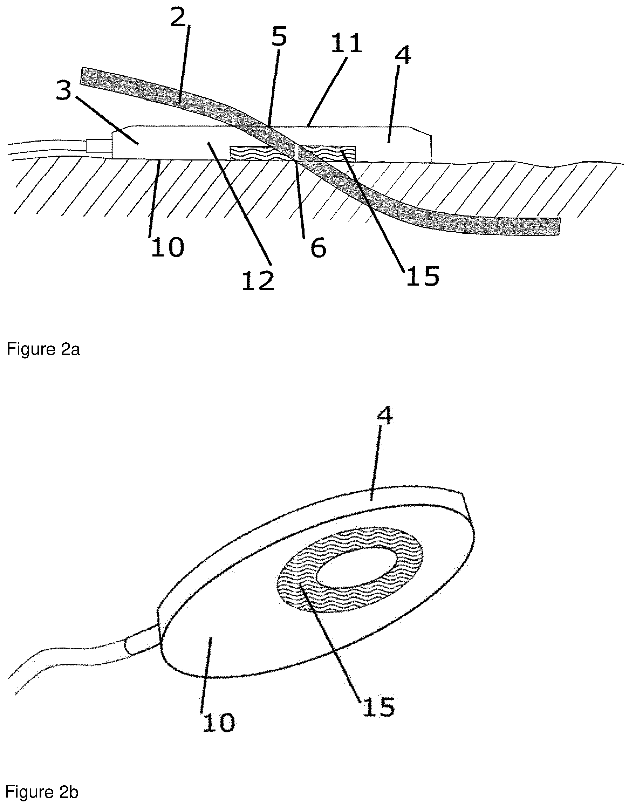

Figures 2a-2d show an embodiment of a system 3 not belonging to the present invention which is similar to the embodiments shown in Fig. 1a-1f. The system 3 illustrated in Fig. 2a-2d also comprises an adaptor 4 having a front outer surface 10, a rear outer surface 11 and a side outer surface 12. Once again, the catheter 2 enters into the adaptor 4 through a catheter entry opening 5 and exits the adapter through a catheter exit opening 6. The catheter 2 is shown in a continuous fashion for illustration purposes even though, from the perspective shown in Fig. 2a, the section of the catheter 2 around which the adaptor 4 is placed cannot normally be seen from this perspective.

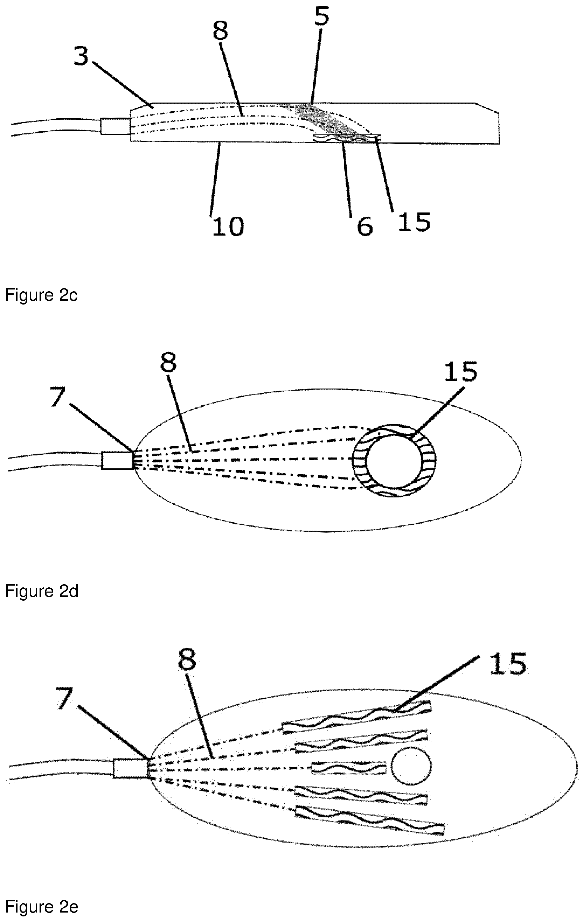

The illustrated system 3 further comprises a diffuser 15, which is ring-shaped and surrounds the catheter exit opening 6, as shown in Fig. 2b. The diffuser 15 is comprised in a lens 16. The illustrated embodiment also comprises light sources 9. The light sources 9 are realized by light emitting ends of light guides 8, as illustrated in Fig. 2c and 2d. The lens 16 forms part of the outer surface of the adaptor 4, specifically of its front outer surface 10. The diffuser 15 is arranged between the light sources 9 and the skin (not shown), such that the UVC light emitted from the light source 9 passes through the diffuser 15.

Figure 2e shows an embodiment of a system 3 not belonging to the present invention, which is similar to the embodiments shown in Fig. 1a-1f. The system 3 shown in figure 2e comprises five rectangular diffuser rods 15.

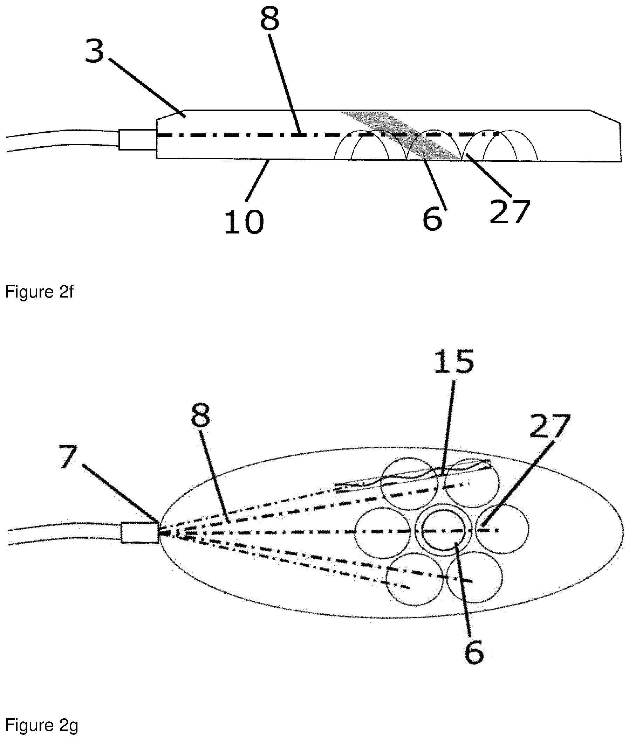

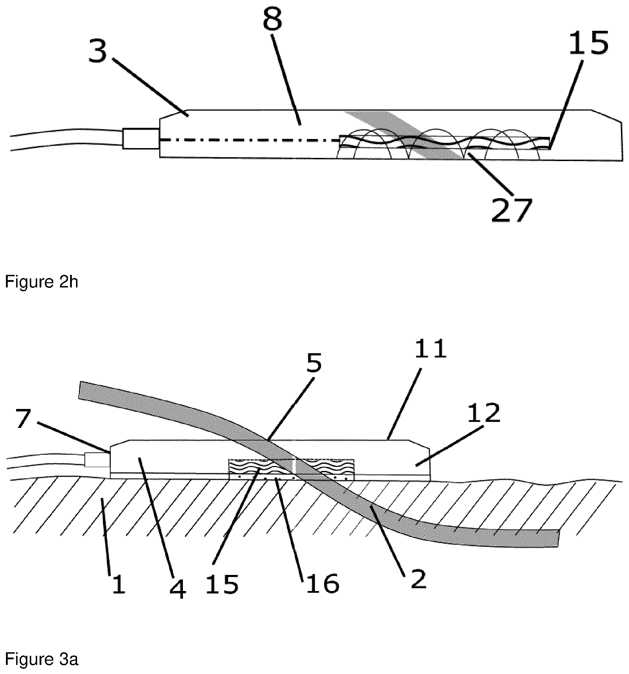

Figures 2f, 2g and 2h show an embodiment of the system 3 disclosed herein which is similar to the embodiments shown in Fig. 1a-1f. The illustrated embodiments include six emission chambers 27. As illustrated in the cross section view in figure 2f, the emission chambers 27 of the illustrated embodiment have a hyperbolic cross section. The emission chambers 27 may further comprise a scattering layer and a filter. As illustrated in the front view illustrated in figure 2g, the emission chambers 27 are arranged symmetrically around the catheter exit opening 6. Each emission chamber 27 comprises one light emitting opening of a light guide 8, such as an optical fiber. Figures 2f, 2g and 2h also illustrate that the system 3 may comprise none, one or more diffusers 15. As an example, as shown in figure 2g, the system 3 may comprise a diffuser 15.

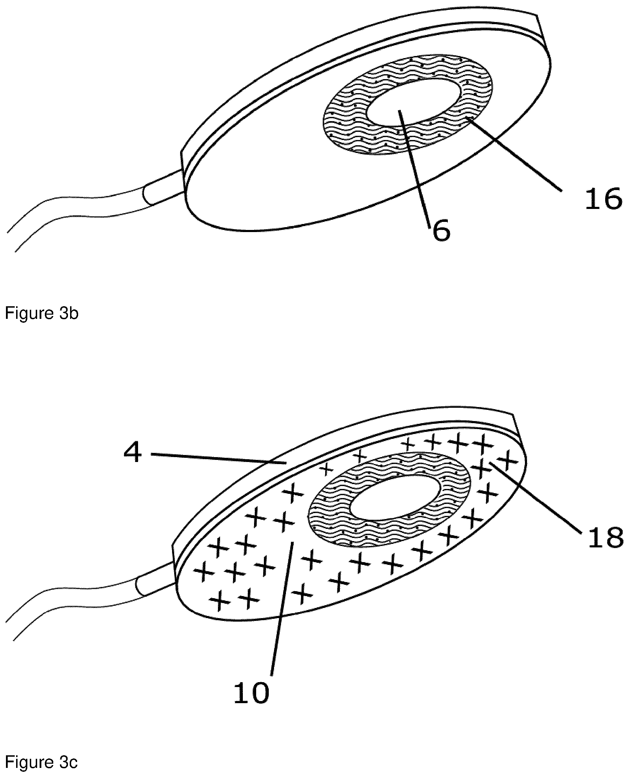

Figures 3a-3b illustrate embodiments of the system 3 disclosed herein featuring a filter The filter is comprised in an emission chamber. Besides the filter, the emission chamber further comprises light sources 9 and a diffuser 15. The embodiment further comprises a lens 16. The emission chamber forms part of the outer surface of the adaptor 4, specifically of its front outer surface 10. The filter and the diffuser 15 are arranged between the light sources 9 and the skin (not shown), such that the UVC light emitted from the light source 9 passes through the diffuser 15.

Figure 3c illustrates a similar embodiment of the system 3 as the one illustrated in Fig. 3a-3b, but further comprises an antimicrobial coating 18. The antimicrobial coating is realized by way of an antimicrobial patch, which is releasably attached to the front outer surface 10 of the adaptor 4.

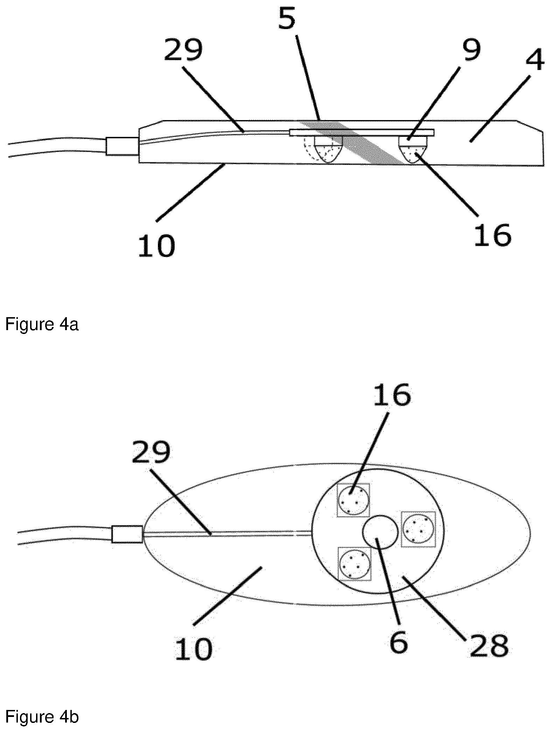

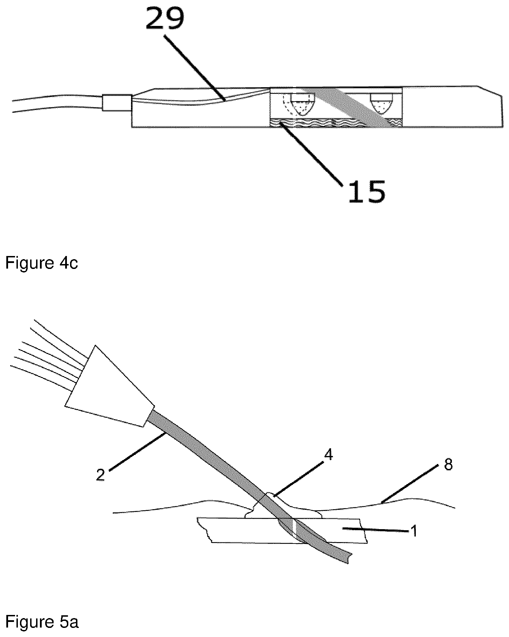

Figures 4a-4c illustrate an embodiment of a system 3 not belonging to the present invention comprising LEDs as light sources 9. The illustrated embodiment comprises three LEDs, each having a diameter of 35 mm. The LEDs are arranged uniformly around the catheter exit opening. The LEDs are comprised in an emission chamber, which also includes a lens 16. The emission chamber forms part of the front outer surface 10 of the adaptor 4. The illustrated embodiment further comprises
a power cable 29 for supplying the LEDs with electrical power. The power cable 29 is connected directly to the LEDs or to the PCB 28. The illustrated embodiment further comprises a lens 16. Further illustrated is the catheter entry opening 5. The embodiment further comprises a printed circuit board 28.

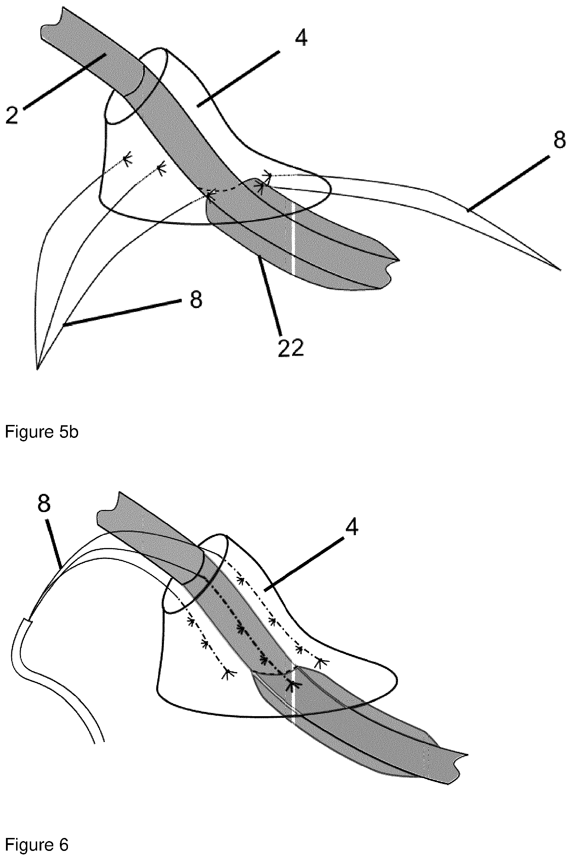

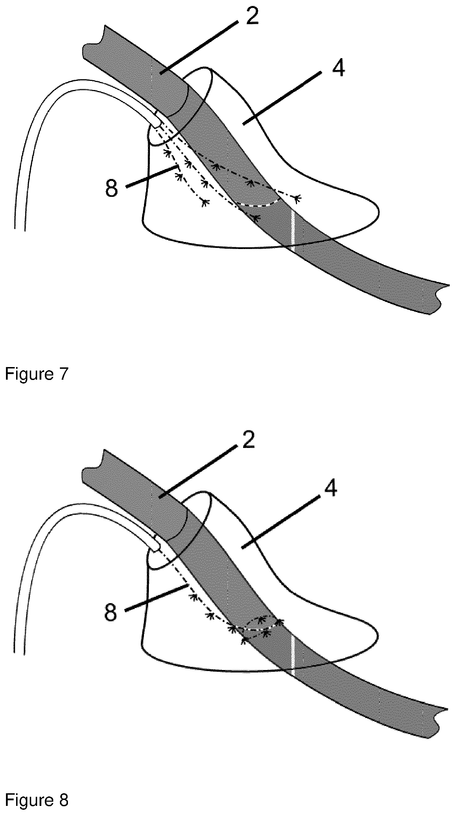

Figures 5-14 relate primarily to embodiments of a system not belonging to the present invention. The adaptor of the system is constructed for medical catheters and tubes that are inserted into the patient for different purposes such as intravenous delivery of drugs, chest drainage, dialysis and air ventilation among others. Within the adaptor, light guides such as optical fibers are placed that target directly the infection-prone sites. This refers to the skin around and within the insertion hole and the tube by shining in situ Far-UVC light (200 nm - 230 nm) for disinfection. Those optical fibers can deliver light by end-point-emitting or side-emitting designs.

The adaptor itself is placed around the catheter before, during or after the insertion process. A coupling mechanism closes the adaptor around the catheter. Eventually, it will be fixed and placed close to or directly at the human skin.

The UVC light source, such as a laser or excimer among others, is fixed at a bed or other infrastructure and emits light in specific intervals through light guides to the adaptor.

The intensity level, the duration and the time interval of the application can vary with the different catherization processes and the state of the patient. Once it is coupled and the light guides in the adaptor are connected to the UVC source, the
disinfection is performed automatically or manually. Moreover, multiple adaptors of different catheters or tubes can be applied simultaneously with the same UVC source.

This invention primarily concerns intravenous catheters including peripherally inserted central catheters (PICC), but it can also be implemented for other medical devices such as chest drainage tubes, indwelling pleural catheters (IPCs), urinary tract catheter, long-term and short-term hemodialysis catheter, peritoneal dialysis, external ventricular drainage (EVD) tube, endotracheal tubes, diabetes procedures and artificial stoma procedures, among others.

The Far-UVC disinfection targets different types of bacteria present in the environment such as Methicilin-resistant Staphylococcus aureus (MRSA), Escherichia coli, Staphylococcus aureus, Staphylococcus epidermis, Salmonella enteritidis, Enterococcus, Bacillus subtilis, Pseudomonas aeruginosa and fungal pneumonia among others.

**Fig. 5** is an embodiment of an adaptor 4, which is attached onto the patient's skin 1, through which a catheter 2 is inserted into the body. The optical fibers as light guides 8 deliver UVC into the adaptor, whereas the end-tips of the in-built optical fibers 8 illuminate the human skin uniformly around the insertion point. The opposite ends of the light guides are then connected to a UVC light source such as a laser or excimer. This UVC light source is capable of generating UVC of a desired wavelength. The UVC light source can be switched on and off at regular intervals of time, depending on the disinfection therapy requirements. A microcontroller for instance can be used to automate this process.

**Fig.** 6 / **Fig.** 7 / **Fig.** 8 depict different concepts of arrangements of optical fibers 8 in the adaptor 4, which can be end-point-emitting, side-emitting and can be coiled (or a combination thereof) within the adaptor around the catheter 2.

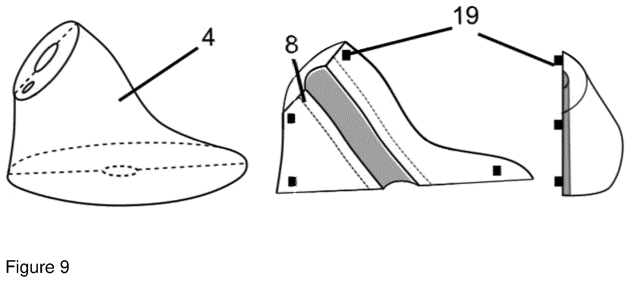

Fig. 9 depicts one concept of a coupling mechanism for the adaptor 4. The physician would then be able to decide when to close the adaptor (before, during or after the insertion of the catheter) with a locking mechanism 19.

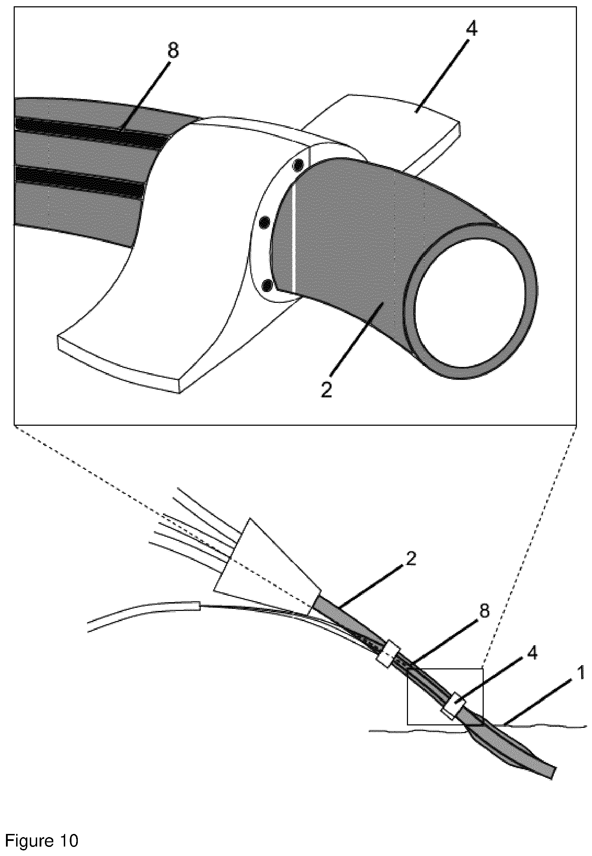

Fig. 10 depicts a second version of an adaptor 4 attached to the outer surface of a catheter 2 near the insertion hole of the skin 1, through which the catheter is inserted into the patient's body. The adaptor has a flat-bottom surface that can be affixed to the patient's body 1 via an adhesive material or a strap-on belt to stabilise the catheter. The adaptor 4 is fed with optical fibers 8, preferably solarization resistant optical fibers with increased durability against UVC radiation. Multiple optical fibers are grouped together into a fiber bundle, and are mechanically coupled to an UVC light source, such as a laser or an excimer, among others.

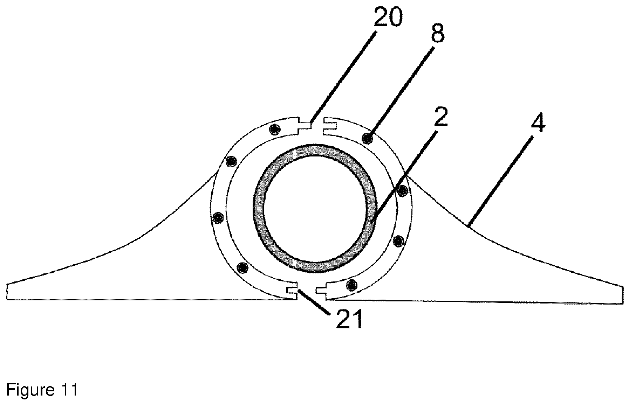

Fig. 11 shows a cross-sectional view of the second version adaptor and the catheter assembly, where multiple end-tips of optical fibers 8 are distributed evenly in this adaptor 4. The adaptor is composed of two symmetric units, which can be clipped together at the coupling points 20 and 21. This allows easy plug-and-play incorporation with any catheter or tube. The end-tips of the fibers disperse UVC radiation such that the area of the patient's skin around the insertion hole, along with the outer surface of the catheter 2, is illuminated. This way, the UVC radiation is able to disinfect all the critical regions near the insertion hole.

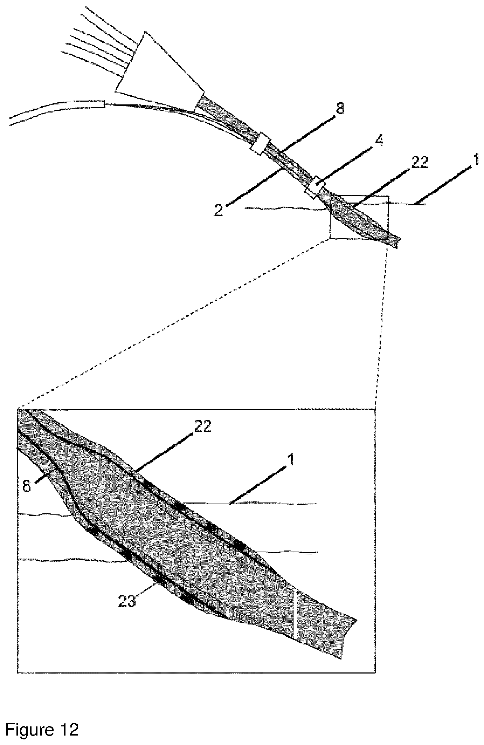

**Fig.** 12 describes an add-on insertion unit 22 of the adaptor 4 that goes into the body, which is involved in the internal disinfection of the epidermis tissues surrounding the catheter. This insertion unit 22 contains in-built optical fibers 8 that are side-emitting 23, whereby the UVC is dispersed radially. In this manner, the UVC radiation is able to illuminate the internal human tissues around the catheter, thereby restricting the further entry of pathogens into the patient's body.

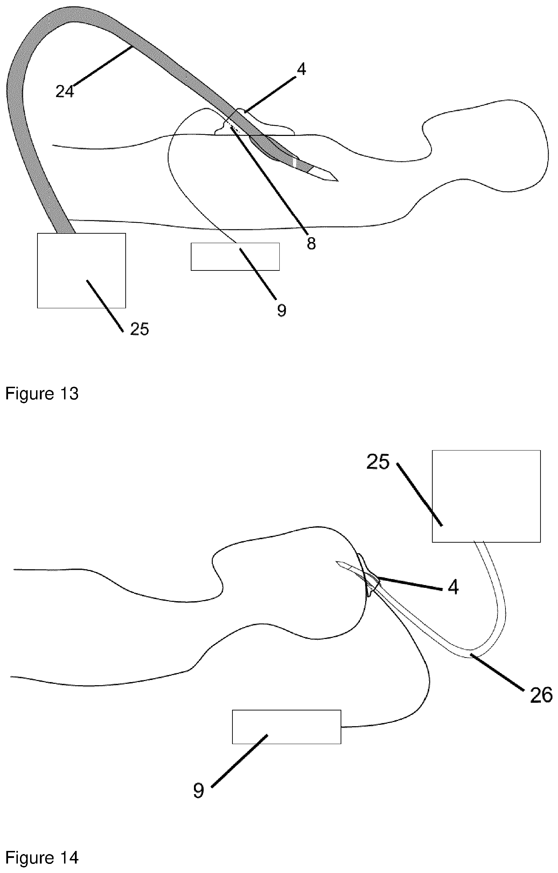

Fig. 13 illustrates an adaptor 4 for a chest drainage tube 24 connected to a drainage system 25, which is attached onto the patient's skin. The optical fibers 8 deliver UVC into the adaptor, whereas the end-tips of these in-built optical fibers 8 illuminate the human skin uniformly around the insertion point. The opposite ends of the light guides are then connected to a UVC light source 9 such as a laser or excimer.

The words used in the specification are words of description rather than limitation. It is understood that various changes may be made without departing from the spirit and scope of the invention.

Fig. 14 illustrates an adaptor 4 for an external ventricular drainage tube 26, which is inserted into the skull of a patient.

### LIST OF DESIGNATIONS

- **1**: skin
- **2**: catheter
- **3**: system
- **4**: adaptor
- **5**: catheter entry opening
- **6**: catheter exit opening
- **7**: light guide entry opening
- **8**: light guide
- **9**: light source
- **10**: front outer surface
- **11**: rear outer surface
- **12**: side outer surface
- **13**: inner surface
- **14**: inner cavity
- **15**: diffuser
- **16**: lens
- **18**: antimicrobial coating
- **19**: locking mechanism
- **20**: first coupling point
- **21**: second coupling point
- **22**: insertion unit
- **23**: side emission
- **24**: chest drainage tube
- **25**: drainage system
- **26**: external ventricular drainage tube
- **27**: emission chamber
- **28**: printed circuit board (PCB)
- **29**: power cable

## Claims

1. System (3) for disinfecting skin tissue around catheters (2), the system (3) comprising an adaptor (4), wherein the adaptor (4) defines a catheter entry opening (5) for entry of a catheter (2) into the adaptor (4) and a catheter exit opening (6) for exit of the catheter (2) from the adaptor (4), wherein the adaptor (4) comprises an outer surface and an inner surface (13) which defines an inner cavity (14) for receiving at least a section of the catheter (2), wherein the inner cavity (14) extends from the catheter entry opening (5) to the catheter exit opening (6);
wherein the system (3) further comprises at least one light source (9) for emitting UVC light, wherein the light source (9) is arranged such that the UVC light is emitted away from and optionally within the adaptor (4);
wherein the system (3) further comprises a light guide (8), which is configured for guiding UVC light from a light receiving opening of the light guide (8) to at least one light emitting opening of the light guide (8), wherein the at least one light source (9) includes a light emitting opening of the light guide (8);
wherein the system comprises at least one emission chamber (27), comprising at least one of the light sources (9); and
wherein the emission chamber (27) comprises the light emitting opening of the at least one light guide (8) as the at least one light source (9);
**characterized in that**
the at least one emission chamber (27) forms part of the outer surface of the adaptor (4) and is arranged around the catheter exit opening (6).

2. System (3) according to claim 1, wherein the outer surface of the adaptor (4) defines a recess located around the catheter exit opening (6), and the emission chamber (27) is arranged in the recess.

3. System (3) according to one of the previous claims, wherein the adaptor (4) comprises a light guide entry opening (7) for entry of the light guide (8) into the adaptor (4), the light guide entry opening (7) being arranged in a side outer surface of the adaptor (4).

4. System (3) according to one of the previous claims, wherein the light guide (8) is an optical fiber, in particular a side-emitting optical fiber.

5. System (3) according to one of the previous claims, wherein the at least one light source (9) is configured to emit UVC light at a wavelength in the range from 200 nm to 230 nm, in particular at a radiant flux of at least 100 µW, preferably from 400 µW to 25000 µW.

6. System (3) according to one of the previous claims, wherein the system further comprises a filter and/or a diffuser (15), wherein the filter and/or the diffuser (15) are arranged such that the UVC light emitted from the light source (9) passes through the filter and/or diffuser (15).

7. System (3) according to claim 6, wherein the system comprises a diffuser (15) which is ring-shaped and surrounds the catheter exit opening (6).

8. System (3) according to one of the previous claims, wherein a front exterior surface of the emission chamber (27) has an area from 1 mm² to 4 cm², preferably from 3 mm² to 2 cm².

9. System (3) according to one of the previous claims, wherein the emission chamber (27) has a thickness from 1 mm to 30 mm, preferably from 5 mm to 15 mm.

10. System (3) according to one of the previous claims, wherein the at least one light source (9) is arranged within a ring surrounding the catheter exit opening (6), the catheter exit opening (6) in particular being circular, a center point of the catheter exit opening (6) being identical with a center point of the ring surrounding the catheter exit opening (6);

11. System (3) according to one of the previous claims, wherein the at least one emission chamber (27)
has an inner surface which is at least partially covered with a scattering layer configured to scatter and reflect UVC light, wherein the scattering layer in particular comprises one of the following materials;
a) polytetrafluoroethylene;
b) aluminium;
c) UV phosphor.

12. System (3) according to one of the previous claims, wherein the outer surface of the adaptor (4) comprises a front outer surface (10) surrounding the catheter exit opening (6), a rear outer surface (11) opposite the front outer (10) surface and a side outer surface (12) arranged between the front outer surface (10) and the rear outer surface (11), wherein the light source (9) is configured such that light is emitted away from the front outer surface (10).

13. System (3) according to claim 12, wherein the front outer surface is covered with a coating (18), wherein preferably the coating (18) is comprised by an adhesive patch and wherein most preferably the adhesive patch has a shape that is identical to a shape of the front outer surface.

14. System (3) according to one of the previous claims, wherein the adaptor (4) comprises a coupling mechanism for closing the adaptor (4) around the catheter (2).

15. System (3) according to one of the previous claims, wherein the adaptor (4) comprises a first adaptor portion and a second adaptor portion which are releasably connectable to each other, preferably in a form-fit fashion, wherein preferably the first adaptor portion and the second adaptor portion each have a connecting surface that intersects the cavity of the adaptor (4), such that the first adaptor portion comprises a first section of the inner surface (13) of the adaptor (4) and the second adaptor portion comprises a second section of the inner surface (13) of the adaptor (4).

16. System (3) according to one of the previous claims, further comprising a guide element for securing and guiding the catheter (2), wherein the guide element comprises an essentially planar resting surface for resting the guide element on a skin (1), and a ring-shaped jacket for securing the catheter (2), wherein the ring-shaped jacket is openable.

17. System (3) according to one of the previous claims, comprising multiple adaptors (4) for different catheters (2), the adaptors (4) being applied simultaneously to a same UVC light source (9).

18. Catheter assembly comprising a catheter (2) and a system (3) according to one of the previous claims. wherein the catheter (2) is inserted into the adaptor (4) such that the catheter (2) extends through the emission chamber (27).

## Patentansprüche

1. System (3) zum Desinfizieren von Hautgewebe um Katheter (2) herum, wobei das System (3) einen Adapter (4) umfasst, wobei der Adapter (4) eine Kathetereintrittsöffnung (5) für den Eintritt eines Katheters (2) in den Adapter (4) und eine Katheteraustrittsöffnung (6) für den Austritt des Katheters (2) aus dem Adapter (4) definiert, wobei der Adapter (4) eine Außenfläche und eine Innenfläche (13) umfasst, die einen inneren Hohlraum (14) zum Aufnehmen mindestens eines Abschnitts des Katheters (2) definiert, wobei sich der innere Hohlraum (14) von der Kathetereintrittsöffnung (5) zur Katheteraustrittsöffnung (6) erstreckt;
wobei das System (3) ferner mindestens eine Lichtquelle (9) zum Emittieren von UVC-Licht umfasst, wobei die Lichtquelle (9) derart angeordnet ist, dass das UVC-Licht vom Adapter (4) weg und optional innerhalb des Adapters (4) emittiert wird;
wobei das System (3) weiter einen Lichtleiter (8) umfasst, der zum Leiten von UVC-Licht von einer Lichtempfangsöffnung des Lichtleiters (8) zu mindestens einer Lichtemissionsöffnung des Lichtleiters (8) konfiguriert ist, wobei die mindestens eine Lichtquelle (9) eine Lichtemissionsöffnung des Lichtleiters (8) umfasst;
wobei das System mindestens eine Emissionskammer (27) umfasst, die mindestens eine der Lichtquellen (9) aufweist; und
wobei die Emissionskammer (27) die Lichtemissionsöffnung des mindestens einen Lichtleiters (8) als die mindestens eine Lichtquelle (9) umfasst;
**dadurch gekennzeichnet, dass**
die mindestens eine Emissionskammer (27) einen Teil der Außenfläche des Adapters (4) bildet und um die Katheteraustrittsöffnung (6) herum angeordnet ist.

2. System (3) nach Anspruch 1, wobei die Außenfläche des Adapters (4) eine Aussparung definiert, die um die Katheteraustrittsöffnung (6) herum angeordnet ist, und die Emissionskammer (27) in der Aussparung angeordnet ist.

3. System (3) nach einem der vorhergehenden Ansprüche, wobei der Adapter (4) eine Lichtleitereintrittsöffnung (7) zum Eintritt des Lichtleiters (8) in den Adapter (4) umfasst, wobei die Lichtleitereintrittsöffnung (7) in einer seitlichen Außenfläche des Adapters (4) angeordnet ist.

4. System (3) nach einem der vorhergehenden Ansprüche, wobei der Lichtleiter (8) eine optische Faser, insbesondere eine seitlich emittierende optische Faser, ist.

5. System (3) nach einem der vorhergehenden Ansprüche, wobei die mindestens eine Lichtquelle (9) konfiguriert ist, um UVC-Licht mit einer Wellenlänge im Bereich von 200 nm bis 230 nm, insbesondere mit einem Strahlungsfluss von mindestens 100 µW, vorzugsweise von 400 µW bis 25000 µW, zu emittieren.

6. System (3) nach einem der vorhergehenden Ansprüche, wobei das System ferner einen Filter und/oder einen Diffusor (15) umfasst, wobei der Filter und/oder der Diffusor (15) derart angeordnet sind, dass das von der Lichtquelle (9) emittierte UVC-Licht durch den Filter und/oder durch den Diffusor (15) hindurchgeht.

7. System (3) nach Anspruch 6, wobei das System einen Diffusor (15) umfasst, der ringförmig ist und die Katheteraustrittsöffnung (6) umgibt.

8. System (3) nach einem der vorhergehenden Ansprüche, wobei eine vordere Außenfläche der Emissionskammer (27) eine Fläche von 1 mm² bis 4 cm², vorzugsweise von 3 mm² bis 2 cm², aufweist.

9. System (3) nach einem der vorhergehenden Ansprüche, wobei die Emissionskammer (27) eine Dicke von 1 mm bis 30 mm, vorzugsweise von 5 mm bis 15 mm, aufweist.

10. System (3) nach einem der vorhergehenden Ansprüche, wobei die mindestens eine Lichtquelle (9) innerhalb eines die Katheteraustrittsöffnung (6) umgebenden Rings angeordnet ist, wobei die Katheteraustrittsöffnung (6) insbesondere kreisförmig ist, wobei ein Mittelpunkt der Katheteraustrittsöffnung (6) mit einem Mittelpunkt des die Katheteraustrittsöffnung (6) umgebenden Rings identisch ist.

11. System (3) nach einem der vorhergehenden Ansprüche, wobei die mindestens eine Emissionskammer (27) eine Innenfläche aufweist, die mindestens teilweise mit einer Streuschicht bedeckt ist, die konfiguriert ist, um UVC-Licht zu streuen und zu reflektieren, wobei die Streuschicht insbesondere eines der folgenden Materialien umfasst:
a) Polytetrafluorethylen;
b) Aluminium;
c) UV-Leuchtstoff.

12. System (3) nach einem der vorhergehenden Ansprüche, wobei die Außenfläche des Adapters (4) eine die Katheteraustrittsöffnung (6) umgebende vordere Außenfläche (10), eine der vorderen Außenfläche (10) gegenüberliegende hintere Außenfläche (11) und eine zwischen der vorderen Außenfläche (10) und der hinteren Außenfläche (11) angeordnete seitliche Außenfläche (12) umfasst, wobei die Lichtquelle (9) derart konfiguriert ist, dass Licht von der vorderen Außenfläche (10) weg emittiert wird.

13. System (3) nach Anspruch 12, wobei die vordere Außenfläche mit einer Beschichtung (18) bedeckt ist, wobei die Beschichtung (18) vorzugsweise von einem Klebepflaster umfasst ist und wobei das Klebepflaster am meisten bevorzugt eine Form aufweist, die mit einer Form der vorderen Außenfläche identisch ist.

14. System (3) nach einem der vorhergehenden Ansprüche, wobei der Adapter (4) einen Kopplungsmechanismus zum Schließen des Adapters (4) um den Katheter (2) herum umfasst.

15. System (3) nach einem der vorhergehenden Ansprüche, wobei der Adapter (4) einen ersten Adapterabschnitt und einen zweiten Adapterabschnitt umfasst, die lösbar miteinander verbindbar sind, vorzugsweise in einer formschlüssigen Weise, wobei der erste Adapterabschnitt und der zweite Adapterabschnitt vorzugsweise jeweils eine Verbindungsfläche aufweisen, die den Hohlraum des Adapters (4) schneidet, derart, dass der erste Adapterabschnitt einen ersten Abschnitt der Innenfläche (13) des Adapters (4) umfasst und der zweite Adapterabschnitt einen zweiten Abschnitt der Innenfläche (13) des Adapters (4) umfasst.

16. System (3) nach einem der vorhergehenden Ansprüche, ferner umfassend ein Führungselement zum Sichern und Führen des Katheters (2), wobei das Führungselement eine im Wesentlichen ebene Auflagefläche zum Auflegen des Führungselements auf eine Haut (1) und einen ringförmigen Mantel zum Sichern des Katheters (2) umfasst, wobei der ringförmige Mantel geöffnet werden kann.

17. System (3) nach einem der vorhergehenden Ansprüche, umfassend mehrere Adapter (4) für verschiedene Katheter (2), wobei die Adapter gleichzeitig an derselben UVC-Lichtquelle (9) angebracht sind.

18. Katheteranordnung, umfassend einen Katheter (2) und ein System (3) nach einem der vorhergehenden Ansprüche, wobei der Katheter (2) derart in den Adapter (4) eingesetzt ist, dass sich der Katheter (2) durch die Emissionskammer (27) erstreckt.

## Revendications

1. Système (3) pour désinfecter du tissu cutané autour de cathéters (2), système (3) comprenant un adaptateur (4), dans lequel l'adaptateur (4) définit une ouverture d'entrée de cathéter (5) pour l'entrée d'un cathéter (2) dans l'adaptateur (4) et une ouverture de sortie de cathéter (6) pour la sortie du cathéter (2) de l'adaptateur (4), dans lequel l'adaptateur (4) comprend une surface extérieure et une surface intérieure (13) qui définit une cavité intérieure (14) pour recevoir au moins une section du cathéter (2), dans lequel la cavité intérieure (14) s'étend de l'ouverture d'entrée de cathéter (5) à l'ouverture de sortie de cathéter (6) ;
dans lequel le système (3) comprend en outre au moins une source lumineuse (9) pour émettre de la lumière UVC, dans lequel la source lumineuse (9) est disposée de telle manière que la lumière UVC est émise à l'extérieur et, en option, à l'intérieur de l'adaptateur (4) ;
dans lequel le système (3) comprend en outre un guide optique (8), qui est configuré pour guider de la lumière UVC d'une ouverture recevant la lumière du guide optique (8) vers au moins une ouverture émettant la lumière du guide optique (8), dans lequel l'au moins une source lumineuse (9) inclut une ouverture émettant la lumière du guide optique (8) ;
dans lequel le système comprend au moins une chambre d'émission (27), comprenant au moins une des sources lumineuses (9) ; et
dans lequel la chambre d'émission (27) comprend l'ouverture émettant la lumière de l'au moins un guide optique (8) comme l'au moins une source lumineuse (9) ; **caractérisé en ce que**
l'au moins une chambre d'émission (27) forme une partie de la surface extérieure de l'adaptateur (4) et est disposée autour de l'ouverture de sortie de cathéter (6).

2. Système (3) selon la revendication 1, dans lequel la surface extérieure de l'adaptateur (4) définit un creux situé autour de l'ouverture de sortie de cathéter (6), et la chambre d'émission (27) est disposée dans le creux.

3. Système (3) selon l'une des revendications précédentes, dans lequel l'adaptateur (4) comprend une ouverture d'entrée de guide optique (7) pour l'entrée du guide optique (8) dans l'adaptateur (4), l'ouverture d'entrée de guide optique (7) étant disposée dans une surface extérieure latérale de l'adaptateur (4).

4. Système (3) selon l'une des revendications précédentes, dans lequel le guide optique (8) est une fibre optique, en particulier une fibre optique à émission latérale.

5. Système (3) selon l'une des revendications précédentes, dans lequel l'au moins une source lumineuse (9) est configurée pour émettre de la lumière UVC à une longueur d'onde comprise entre 200 nm et 230 nm, en particulier à un flux rayonnant d'au moins 100 µW, de préférence entre 400 µW et 25000 µW.

6. Système (3) selon l'une des revendications précédentes, dans lequel le système comprend en outre un filtre et/ou un diffuseur (15), dans lequel le filtre et/ou le diffuseur (15) est disposé de telle manière que la lumière UVC émise par la source lumineuse (9) traverse le filtre et/ou le diffuseur (15).

7. Système (3) selon la revendication 6, dans lequel le système comprend un diffuseur (15) qui a une forme annulaire et entoure l'ouverture de sortie de cathéter (6).

8. Système (3) selon l'une des revendications précédentes, dans lequel une surface extérieure avant de la chambre d'émission (27) a une surface entre 1 mm² et 4 cm², de préférence entre 3 mm² et 2 cm².

9. Système (3) selon l'une des revendications précédentes, dans lequel la chambre d'émission (27) a une épaisseur entre 1 mm et 30 mm, de préférence entre 5 mm et 15 mm.

10. Système (3) selon l'une des revendications précédentes, dans lequel l'au moins une source lumineuse (9) est disposée à l'intérieur d'un anneau entourant l'ouverture de sortie de cathéter (6), l'ouverture de sortie de cathéter (6) en particulier étant circulaire, un point central de l'ouverture de sortie de cathéter (6) étant identique à un point central de l'anneau entourant l'ouverture de sortie de cathéter (6).

11. Système (3) selon l'une des revendications précédentes, dans lequel l'au moins une chambre d'émission (27) a une surface intérieure qui est au moins partiellement recouverte d'une couche de dispersion, configurée pour disperser et réfléchir la lumière UVC, dans lequel la couche de dispersion en particulier comprend un des matériaux suivants :
a) polytétrafluoroéthylène ;
b) aluminium ;
c) phosphore UV.

12. Système (3) selon l'une des revendications précédentes, dans lequel la surface extérieure de l'adaptateur (4) comprend une surface extérieure avant (10) entourant l'ouverture de sortie de cathéter (6), une surface extérieure arrière (11) en face de la surface extérieure avant (10) et une surface extérieure latérale (12) disposée entre la surface extérieure avant (10) et la surface extérieure arrière (11), dans lequel la source lumineuse (9) est configurée de telle sorte que de la lumière est émise à l'extérieur de la surface extérieure avant (10).

13. Système (3) selon la revendication 12, dans lequel la surface extérieure avant est recouverte d'un revêtement (18),
dans lequel, de préférence, le revêtement (18) est constitué d'un patch adhésif et dans lequel, le plus préférablement, le patch adhésif a une forme identique à une forme de la surface extérieure avant.

14. Système (3) selon l'une des revendications précédentes, dans lequel l'adaptateur (4) comprend un mécanisme de couplage pour fermer l'adaptateur (4) autour du cathéter (2).

15. Système (3) selon l'une des revendications précédentes, dans lequel l'adaptateur (4) comprend une première portion d'adaptateur et une seconde portion d'adaptateur qui peuvent être reliées entre elles de manière amovible, de préférence en s'emboîtant, dans lequel, de préférence, la première portion d'adaptateur et la seconde portion d'adaptateur ont chacune une surface de connexion qui coupe la cavité de l'adaptateur (4), de manière à ce que la première portion d'adaptateur comprend une première section de la surface intérieure (13) de l'adaptateur (4) et la seconde portion d'adaptateur comprend une seconde section de la surface intérieure (13) de l'adaptateur (4).

16. Système (3) selon l'une des revendications précédentes, comprenant en outre un élément de guidage pour maintenir et guider le cathéter (2), dans lequel l'élément de guidage comprend une surface d'appui essentiellement plane pour appuyer l'élément de guidage sur une peau (1), et une gaine en forme d'anneau pour maintenir le cathéter (2), dans lequel la gaine en forme d'anneau peut être ouverte.

17. Système (3) selon l'une des revendications précédentes, comprenant de multiples adaptateurs (4) pour différents cathéters (2), les adaptateurs (4) étant appliqués simultanément à une même source de lumière UVC (9).

18. Assemblage de cathéters comprenant un cathéter (2) et un système (3) selon l'une des revendications précédentes,
dans lequel le cathéter (2) est inséré dans l'adaptateur (4) de manière à ce que le cathéter (2) s'étende à travers la chambre d'émission (27).
